# EUROPEAN PATENT APPLICATION

(11) **EP 4 410 969 A1**
(43) Date of publication of application: **07.08.2024**
(21) Application number: 22876926.1
(22) Date of filing: 29.09.2022
(51) Int. Cl.: C12N 7/00, C07K 14/005, A61K 39/155, A61K 35/76, A61P 31/12

(54) **RECOMBINANT LIVE ATTENUATED RSV VACCINE STRAIN AND PRODUCTION METHOD THEREFOR**

(30) Priority: 29.09.2021 KR 20210129272; 19.07.2022 KR 20220089158
(71) Applicant: SK Bioscience Co., Ltd., Bundang-gu, Seongnam-si, Gyeonggi-do, 13494 (KR)
(72) Inventor: SEO, Ki-weon, Seongnam-si, Gyeonggi-do 13494 (KR); KWON, Teawoo, Seongnam-si, Gyeonggi-do 13494 (KR); KIM, Eun-som, Seongnam-si, Gyeonggi-do 13494 (KR)
(74) Representative: Engelhard, Markus
(86) International application number: PCT/KR2022/014712
(87) International publication number: WO 2023/055154

(57) **Abstract**

The present invention provides a recombinant attenuated respiratory syncytial virus (RSV) comprising i) a nucleic acid encoding an F protein of a stabilized pre-fusion respiratory syncytial virus (RSV) or its analogue, variant, or fragment; or ii) a nucleic acid encoding a G protein of vesicular stomatitis Indiana virus (VSV) or its analogue, variant, or fragment, and provides a genome of the recombinant RSV, and a recombinant vector comprising the genome. The recombinant attenuated RSV can be provided as a live vaccine strain which is safe and has excellent stability while maintaining infectiousness.

## Description

### TECHNICAL FIELD

The present application claims the priority based on Korean application No. 10-2021-0129272 filed on September 29, 2021 and Korean application No. 10-2022-0089158 filed on July 19, 2022, and the entire contents disclosed in the description and drawings of the corresponding application are incorporated herein. The present invention relates to a recombinant attenuated RSV, a method for preparing the same, or a vaccine comprising the same, and more specifically, it relates to a recombinant attenuated RSV capable of producing a live vaccine strain with excellent stability and safety, a method for preparing the same, or a vaccine comprising the same.

### BACKGROUND ART

Respiratory Syncytial Virus (RSV) is a virus that is widely prevalent all around the world, and is a virus which causes respiratory disease symptoms, and in particular, is a main cause of severe respiratory infection death in infants. Infants are main targets for infection, but it is known to cause fatal respiratory diseases by causing infection of respiratory diseases of patients with weakened immune systems and the elderly. Although it is the second-highest cause of inducing respiratory disease symptoms after influenza, it is known that the annual mortality rate due to the RSV per 100,000 in infants under 1 year of age is approximately 1.3-2.5 times higher than that of influenza. According to the WHO report in 2002, it was reported that people infected by the RSV was 64 million people every year, and among them, 160,000 people died.

For a purpose of RSV disease prevention, initially, a vaccine using an inactivated virus (inactivated vaccine) was developed, but due to occurrence of serious side effects (ERD (enhanced respiratory disease)) such as inducing disease symptoms more severely and the like, the use of the inactivated vaccine has become impossible.

Accordingly, researchers have tried to develop a vaccine which does not cause ERD and has an excellent ability to induce neutralizing antibodies.

In particular, live vaccines have an advantage in that ERD is not caused and the ability to induce neutralizing antibodies is excellent, but the RSV is very metastable, so considering the stability problem and safety problem of the virus, there were difficulties in developing a vaccine.

RSV belongs to the Orthopneumoviridae subfamily belonging to the Mononegavirales order, Pneumoviridae family. RSV is known to be a medium-sized virus of about 120-200nm. Wild type (wt) RSV genome or antigenome consists of the following 10 genes and 11 virus proteins. The 11 RSV proteins an RNA-binding nucleoprotein (N), a phosphoprotein (P), a large polymerase protein (L), an attachment glycoprotein (G), a fusion protein (F), a small hydrophobic (SH) surface glycoprotein, an internal matrix protein (M), and 2 non-structural proteins, NS1 and NS2, and M2-1 and M2-2 proteins. The complete amino acid sequences of these proteins are known in the art. The RSV gene order is 3'-NS1-NS2-N-P-M-SH-G-F-M2-L. Transcription starts from a single promoter at the 3' end, and proceeds sequentially. The genome of the RSV is a single strand of non-segmented negative sense RNA of 15.2 kb. Herein, the RSV F protein is an important component which initiates virus entry and performs a fusion action with a cell membrane and is known as a major target of a vaccine and antiviral drug due to high antigenicity. However, the F protein antigen is a stabilized form of a pre-fusion protein, and there are problems in that production and purification are difficult and it is difficult to secure stability, and it is not easy to maintain efficacy.

Accordingly, the inventors of the present invention intend to resolve instability of an F protein and develop a safe vaccine using a new type of attenuated RSV.

### DISCLOSURE

### Technical Problem

Accordingly, a problem to be solved by the present invention is to provide a new type of vaccine capable of operating an effective immune system.

A problem to be solved by the present invention is to develop a live RSV vaccine strain with excellent stability and safety.

### Technical Solution

One embodiment provides a recombinant attenuated respiratory syncytial virus comprising a stabilized pre-fusion RSV F protein, or a nucleic acid encoding a G protein of vesicular stomatitis Indiana virus (VSV) or its analogue, variant, or fragment. Preferably, the G protein comprises a recombinant virus consisting of the amino acid sequence of SEQ ID NO: 2.

One embodiment provides a recombinant attenuated respiratory syncytial virus comprising a stability inducing mutation or a safety inducing mutation. In the recombinant respiratory syncytial virus comprising a nucleic acid encoding a G protein of vesicular stomatitis Indiana virus (VSV) or its analogue, variant, or fragment, a nucleic acid encoding at least one selected from the group consisting of SH, G, and F proteins of the RSV may be further i) deleted, or ii) substituted with another nucleic acid, or iii) any one of nucleic acids encoding the proteins is deleted, and the others are substituted with another nucleic acid.

For inducing stability, a mutation that maintains the F protein of RSV in a prefusion form is comprised, or (or, comprised and) a protein consisting of the amino acid sequence represented by SEQ ID NO: 2 or a functional fragment thereof (i.e., chimeric VSV G protein). In order to maintain it in a prefusion form, 1) a mutation which structurally stabilizes the F protein (D486L/E487L/F488W) may be introduced, or 2) it may be maintained in a prefusion form by changing the furin cleavage site of the F protein to control cleavage of the protein.
1) Specifically, it may be manufactured by deleting 514-575 residues corresponding to a transmembrane domain and a cytoplasmic tail and adding a foldon sequence which is a heterogenous trimeric domain to express this mutant F protein into a soluble prefusion trimeric F protein which is not present on the virus surface and is secreted into the outside of the infected cells together with the structural stabilizing mutation (D486L/E487L/F488W). Therefore, this soluble prefusion trimeric F protein may have advantages capable of inducing an immune response without affecting instability of the virus.
2) Specifically, in the mutated F protein, in which at least one of furin cleavage sites of the F protein of the recombinant attenuated respiratory syncytial virus (RSV) is mutated, the peptide of SEQ ID NO: 55 corresponding to furin cleavage site II of an F gene which is the 106~109th amino acid sequences may be modified into the peptide of SEQ ID NO: 56, or the peptide of SEQ ID NO: 57 corresponding to furin cleavage site I of an F gene which is the 133~136th amino acids may be substituted with the peptide of SEQ ID NO: 58. Otherwise, it may be modified to maintain in a single chain form by connecting two furin cleavage sites with a linker sequence (SEQ ID NO: 54) to prevent cleavage. Therefore, the stability of the virus is to be increased by maintaining the F protein in a prefusion form.

In addition, in another embodiment, for increasing safety, a protein (SH or G or F) of the RSV may be deleted or NS1, NS2 genes for inhibiting an immune evasion mechanism may be further deoptimized.

Through a combination of mutations for increasing the stability and safety as above, a recombinant attenuated respiratory syncytial virus is provided.

In one embodiment, the recombinant attenuated respiratory syncytial virus may be provided in a form in which i) at least one protein selected from the group consisting of SH, G and F proteins of RSV is deleted, or ii) at least one protein selected from the group consisting of the SH, G and F proteins is substituted, or iii) at least one protein selected from the group consisting of the SH, G and F proteins is deleted and proteins other than the deleted protein are substituted with a new protein, while comprising a protein consisting of the amino acid sequences represented by SEQ ID NO: 2 or a functional fragment thereof.

In one embodiment, in case of the ii), it may comprise a nucleic acid sequence consisting of SEQ ID NO: 11 or SEQ ID NO: 14, preferably, an amino acid encoding the cDNA sequence.

In other embodiment, in case of the iii), it may comprise a nucleic acid sequence consisting of SEQ ID NO: 12 or SEQ ID NO: 13, preferably, an amino acid sequence encoding the cDNA sequence.

In one embodiment, in a mutated F protein, in which at least one among furin cleavage sites of the F protein of the recombinant attenuated respiratory syncytial virus (RSV) is mutated, the peptide of SEQ ID NO: 55 corresponding to furin cleavage site II of an F gene which is the 106-109th amino acid sequences of the F protein may be modified into the peptide of SEQ ID NO: 56, or the peptide of SEQ ID NO: 57 corresponding to furin cleavage site I of an F gene which is the 133~136th amino acids may be substituted with the peptide of SEQ ID NO: 58. In addition, it may be modified to maintain a single chain form by connecting two furin cleavage sites with a linker sequence to prevent cleavage.

In other embodiment, genes encoding NS 1 and NS2 proteins comprised in the virus may be further substituted, and the antigenomic cDNA of the substituted genes may consist of a nucleotide sequence represented by SEQ ID NOs: 32 and 33, respectively.

One example of the present invention provides an isolated polynucleotide molecule comprising a nucleic acid encoding an F protein of a stabilized pre-fusion respiratory syncytial virus (RSV) or an analogue, variant or fragment thereof; or a nucleic acid encoding a G protein of vesicular stomatitis Indiana virus (VSV) or an analogue, variant, or fragment thereof. The nucleic acid may be antigenomic cDNA or RNA, and preferably, it may be cDNA, and it may be used for co-transfection with an expression vector encoding some of the proteins of the RSV.

One embodiment of the present invention may provide a novel use of the recombinant attenuated RSV or a polynucleotide molecule thereof, for preparing a drug for preventing or treating infection of an RSV.

In one embodiment, the isolated polynucleotide molecule may be cDNA consisting of a polynucleotide represented by at least one selected from the group consisting of SEQ ID NOs: 6 to 16. The cDNA sequence may have sequence identity of at least 70%, at least 80%, at least 85%, at least 90%, at least 91%, at least 92%, at least 93%, at least 94%, at least 95%, at least 96%, at least 97%, at least 98%, at least 99%, or 100% with the at least one polynucleotide sequence selected from the group consisting of SEQ ID NOs: 6 to 16.

One embodiment provides a vector comprising the isolated polynucleotide molecule. The vector may be used for co-transfection with an expression vector encoding N, P, L, M2-ORF1 proteins of RSV.

One embodiment provides a cell comprising an isolated polynucleotide molecule or vector.

One embodiment provides a method for producing a recombinant attenuated RSV comprising the followings.

Specifically, the method may comprise transfecting a vector comprising the isolated polynucleotide molecule into a host cell, culturing the cell or a culture thereof for a sufficient time to allow virus replication, and isolating the replicated recombinant RSV.

One embodiment provides a recombinant attenuated RSV produced by the method.

One embodiment provides a respiratory syncytial virus immunogenic composition comprising a recombinant attenuated RSV.

In one embodiment, the immunogenicity may be a vaccine, inter alia, a live vaccine.

In one embodiment, the immunogenic composition may further comprise at least one selected from the group consisting of a carrier, a diluent, an excipient, and an adjuvant. In one embodiment, the immunogenic composition may be administered through an intranasal, intratracheal, intramuscular, intradermal or subcutaneous route.

One embodiment provides a pharmaceutical composition for preventing or treating infection of a respiratory syncytial virus comprising the immunogenic composition.

One embodiment provides a method for inducing an immune response against an RSV in a subject or a method for preventing infection of a respiratory syncytial virus, comprising administering the pharmaceutical composition in an effective amount to produce immune into a subject in need thereof.

One embodiment provides a use of a recombinant RSV, for inducing an immune response against an RSV in a subject.

One embodiment is a pharmaceutical composition for manufacturing a drug for preventing or treating RSV infection, and the composition may provide a composition comprising a cDNA molecule of a recombinant RSV or a functional fragment or analogue thereof.

Preferably, the drug may comprise a vaccine for preventing RSV infection.

The cDNA molecule of the recombinant RSV may comprise a composition comprising any one selected from the group consisting of SEQ ID NOs: 6 to 16.

The vector includes a T7 promoter, a hammerhead ribozyme, a hepatitis delta virus ribozyme, and a T7 terminator needed to produce a recombinant virus, and may comprise any one cDNA selected from the group consisting of SEQ ID NOs: 17 to 27.

### Advantageous Effects

The present invention provides a live RSV vaccine strain with excellent stability and safety.

The present invention provides a new type of RSV vaccine capable of inducing a defense mechanism against an RSV.

The recombinant attenuated RSV of the present invention can resolve instability of the virus by expressing an F protein in a prefusion form on the virus surface.

The recombinant attenuated RSV of the present invention can resolve instability of the virus by expressing a soluble trimeric F protein in a prefusion form and also induce immune by the F protein.

The recombinant attenuated RSV of the present invention can resolve instability of the virus by allowing VSV G to perform a role of viral infection instead of the F protein.

The recombinant attenuated RSV of the present invention can resolve instability of the virus by removing the F protein.

The recombinant attenuated RSV of the present invention does not produce secreted G, and therefore, inhibits an immune escaping mechanism, and thereby, a new type of vaccine that can operate an effective immune system is provided.

The recombinant attenuated RSV of the present invention can reduce the expression level of NS 1 or NS2 protein, thereby inhibiting an immune evasion mechanism, and it is possible to operating an effective immune system.

The present invention can increase the safety of the vaccine by removing an SH protein or G protein or F protein of RSV to attenuate a recombinant RSV. The present invention provides a new recombinant RSV.

### DESCRIPTION OF DRAWINGS

The following drawings attached to the present description illustrate preferable examples of the present invention, and play a role of further understanding the technical spirit of the present invention together with the invention described above, and the present invention should not be construed as limited only to the matters described in such drawings.

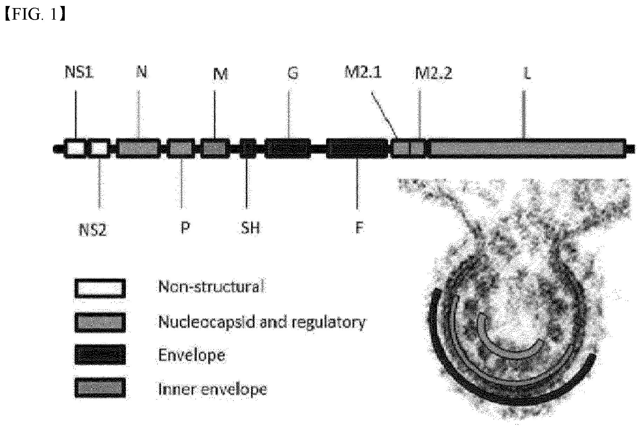

FIG. 1 is a schematic diagram of the genome structure of the RSV and is a drawing which shows the RSV genome and form.

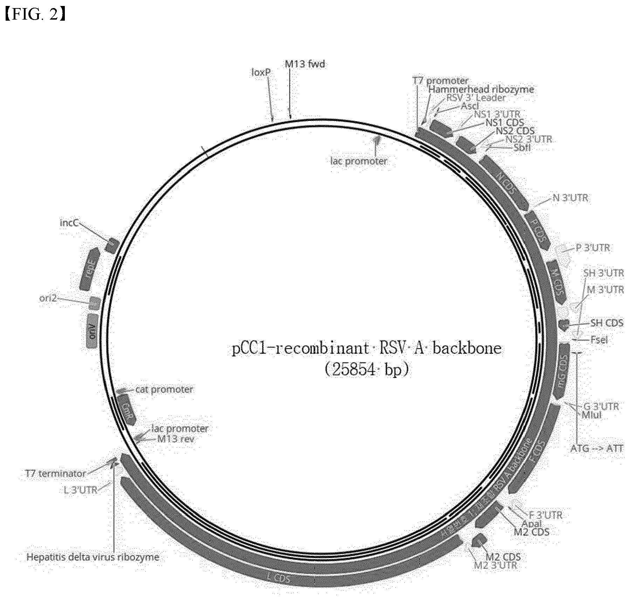

FIG. 2 is a vector map and shows the antigenomic cDNA and cloning vector of the recombinant RSV A backbone.
FIG. 3 is a schematic diagram of the negative-sense RSV genome structure of cRSVA_VSVG_A, and the recombinant chimeric VSV G gene is inserted between the SH gene and G gene of the recombinant RSV A backbone.
FIG. 4 is a schematic diagram of the negative-sense RSV genome structure of cRSVA_VSVG_A_ΔSH, and the SH gene of the recombinant RSV A backbone is deleted and the recombinant chimeric VSV G gene is inserted in front of the G gene.
FIG. 5 is a schematic diagram of the negative-sense RSV genome structure of cRSVA_VSVG_A_ΔSH_ΔG, and the SH gene and G gene of the recombinant RSV A backbone are deleted and the recombinant chimeric VSV G gene is inserted.
FIG. 6 is a schematic diagram of the negative-sense RSV genome structure of cRSVA_VSVG_A_ΔSH_ΔF, and the SH gene and F gene of the recombinant RSV A backbone are deleted and the recombinant chimeric VSV G gene is inserted in front of the G gene.
FIG. 7 is a schematic diagram of the negative-sense RSV genome structure of cRSVAVSVG S, and the F gene of the recombinant RSV A backbone is substituted with the recombinant chimeric VSV G gene.

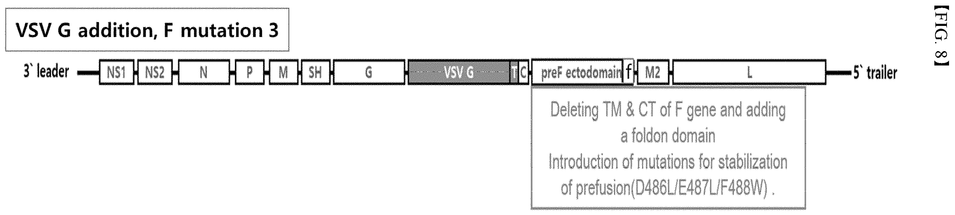

FIG. 8 is a schematic diagram of the negative-sense RSV genome structure of cRSVA_VSVG_A_preF_ef, and the recombinant chimeric VSV G gene is inserted in front of the G gene, and in the F gene, the prefusion stabilizing mutation (D486L, E487L, F488W) and mutation of deleting 514-575 residues, and mutation of inserting the linker and foldon consisting of SEQ ID NO: 54 are inserted. The VSV G performs a role of cell infection instead of the instable RSV F, and the RSV F is inserted in an ectodomain form, and is not expressed on the cell and virus surfaces, so it cannot perform a role of infection, and instead, when cells are infected with the virus, it is expressed in the cells and secreted outside of the cells, thereby inducing only an immune response. In the F ectodomain, a mutation of stabilizing in a prefusion and a trimeric domain are added, so it is expressed into a soluble preF trimer.
FIG. 9 is a schematic diagram of the negative-sense RSV genome structure of cRSVA_VSVG_S_preF_ef, which is a form in which the gene is additionally deleted in FIG. 8 to further attenuate it.
FIG. 10 is a schematic diagram of the negative-sense RSV genome structure of cRSVA_VSVG_S_preF_sc, in which the preF ectodomain-foldon is substituted with a single chain F in FIG. 9. The single chain F can play only a role of inducing immune, as it is expressed on the cell and virus surfaces, but is not modified into a postfusion form, so cannot cause infection. It is expected to induce an immune response much more similar to an actual virus as it is expressed in a state attached to the insoluble cell or virus membrane.
FIG. 11 is a schematic diagram of the negative-sense RSV genome structure of cRSVA_VSVG_A_preF_ef_NS1/NS2deop, in which the DNA sequence encoding NS1 and NS2 proteins in FIG. 8 is deoptimized for human codons. When the expression level is reduced in human cells by this codon deoptimization, the virus is attenuated.
FIG. 12 shows a schematic diagram of the negative-sense RSV genome structure of cRSVA_mF1.
FIG. 13 shows a schematic diagram of the negative-sense RSV genome structure of cRSVA_mF2.

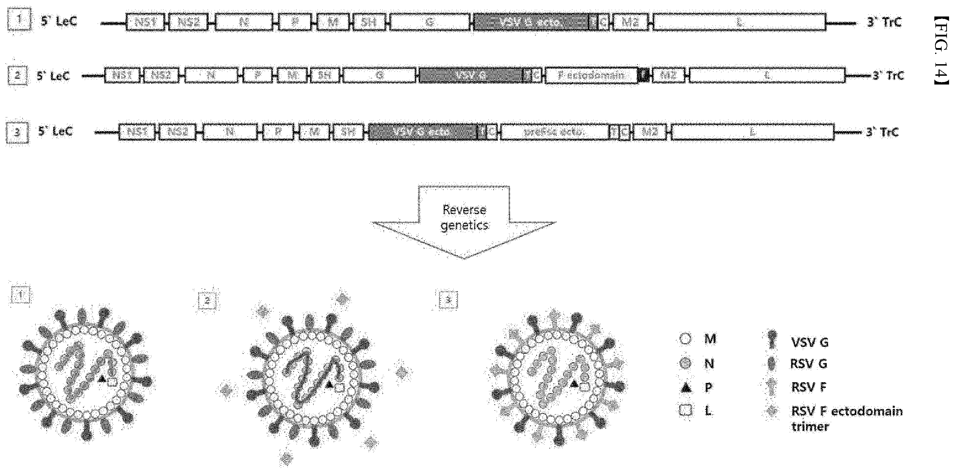

FIG. 14 shows an example of the recombinant RSV recovered from the recombinant RSV antigenomic cDNA through reverse genetics.
FIG. 15 is the result of confirming protein expression into a prefusion form due to the prefusion stabilizing mutation (D486L, E487L, F488W) comprised in SEQ ID NO: 11. It was confirmed by detecting it with a prefusion F-specific antibody (D25).
FIGs. 16 to 20 are results of detecting the virus using IF A analysis method after infecting 10 kinds of viruses rescued by reverse genetics into culture cells.
FIG. 21 is a drawing of comparing plaque patterns of the Backbone strain, Wild type RSV and the recombinant virus prepared in the example.
FIGs. 22 to 24 are results of confirming the stability by temperature against the Backbone strain, Wild type RSV and the recombinant virus prepared in the example (FIG. 22: -80°C; FIG. 23: 4°C; FIG. 24L Room temp.).
FIGs. 25 to 27 are results of analyzing the antibody titer of IgG in serum of mice immunized with the recombinant virus prepared in the example (FIG. 25: Day 14 after first inoculation; FIG. 26: Day 28 after first inoculation; FIG. 27: Antibody titer by antigen).
FIG. 28 is the result of analyzing the neutralizing antibody titer in serum of mice immunized with the recombinant virus prepared in the example.
FIG. 29 is the result of analyzing the virus clearance effect of the recombinant virus prepared in the example.

### BEST MODE

In order to solve the problems, the present invention provides a recombinant attenuated respiratory syncytial virus (RSV) with excellent stability or safety. The RSV mya be preferably used as a live vaccine strain for preventing RSV infection. The recombinant RSV of the present invention may be manufactured using "reverse genetic engineering". Reverse genetic engineering is also called reverse genetics, and it may be used for production of various RNA viruses including a positive (+) stranded RNA virus, a negative (-) stranded RNA virus and a double stranded RNA virus. The present invention provides a method for producing a recombinant virus obtained by using the method of reverse genetics of the present invention.

Reverse genetics may be performed using conventional methods and means already well known to those skilled in the art, and those skilled in the art can obtain a recombinant RSV without difficulties through methods broadly known in the art and the following description. For example, a desired recombinant virus can be produced by converting an amino acid sequence of a target protein or polypeptide into a corresponding nucleic acid sequence, and modifying the nucleic acid sequence into a nucleic acid sequence which can be recognized by a host cell and encode a target protein, synthesizing the modified nucleic acid using primers in vitro to produce a plasmid, and transforming the plasmid. As references, i) Stobart, Christopher C., et al. "BAC-based recovery of recombinant respiratory syncytial virus (RSV)." Reverse Genetics of RNA Viruses. Humana Press, New York, NY, 2017. 111-124., ii) Collins, Peter L., Rachel Fearns, and Barney S. Graham. "Respiratory syncytial virus: virology, reverse genetics, and pathogenesis of disease." Challenges and opportunities for respiratory syncytial virus vaccines (2013): 3-38., iii) Hu, Bing, et al. "Development of a reverse genetics system for respiratory syncytial virus long strain and an immunogenicity study of the recombinant virus." Virology journal 11.1 (2014): 1-16. And the like may be referred.

"Recombinant" may mean a case in which cells, proteins or genes or the like with different genetic traits are present in one organism together, and in particular, "recombinant" used herein refers to a case where a new genetic mutation is generated by inserting genetic information (nucleic acid) from a different subject based on one genetic backbone.

Recombinant RSV (rRSV) refers to an RSV or RSV-like virus which is derived from a recombinant expression system directly or indirectly or is proliferated from a virus or subvirus produced therefrom. The expression construct encoding a virus RNA molecule used for the present invention may be any expression construct commonly used in the art for rescuing a virus. The expression construct may be a vector such as a plasmid or other episome constructs. Such vectors may comprise at least one bacterium and/or eukaryotic replication origin. In addition, the vector may comprise a selective marker. Examples of this selective marker may include genes giving resistance to antibiotics such as chloramphenicol, ampicillin, or kanamycin. The vector may comprise one or more various cloning sites which allow cloning of a DNA sequence.

The recombinant expression system may comprise a recombinant expression vector. "Vector" refers to a DNA construct containing a DNA sequence operably linked to an appropriate regulatory sequence which can perform expression of DNA in a suitable host. It comprises a functionally linked transcription unit, comprising at least one genetic element or combination of elements having a regulatory function in expression of cDNA of an RSV gene, and examples of the elements include a promoter, a structure transcribed to RSV mRNA or a coding sequence, and appropriate transcription initiation and termination sequences. The vector of the present invention may use any vector known in the art, and for example, it may be a plasmid, cosmid, phage particle, or virus vector, and as long as it is can be replicated in a cell, it is not particularly limited thereto.

In the present invention, "recombinant vector" may be used as an expression vector of a target polypeptide capable of expressing the target polypeptide with high efficiency in an appropriate host cell, when an encoding gene of a target polypeptide to be expressed, and the recombinant vector may be expressed in a host cell. Depending on the type of the host cell, an expression regulatory sequence such as a promoter, a terminator, an enhancer and the like, a sequence for membrane targeting or secretion, and the like may be appropriately selected and be variously combined on purposes. In the present invention, the host cell may be preferably a eukaryotic cell, and according to one embodiment of the present invention, it may be a Vero cell, but not limited thereto.

The term of "operably linked" used in the present invention refers to that a gene requiring expression and a regulatory sequence thereof are linked in a method of being combined functionally with each other to enable gene expression.

The method for introducing DNA in a vector form into a cell is not particularly limited, and for example, it may be conducted according to methods fully known to those skilled in the art to which the present invention pertains such as nucleofection, transient transfection, cell fusion, liposome-mediated transfection, polybrene-mediated transfection, transfection using calcium phosphate, transfection by DEAE dextran, transfection by microinjection, transfection by cationic lipids, electroporation, transduction or transfection and the like.

"Genome" used herein is a total base sequence of genes of one organism and means an assembly of all genetic information possessed by one organism. For example, the genome of a virus is used as a meaning encompassing all genetic information sequences of the entire virus.

"Gene" used herein may be understood as a part encoding a protein, and may be DNA or RNA.

The expression used herein, "cDNA or cDNA sequence" means a DNA form of a virus genomic RNA sequence, and this refers to a sequence which differs from an RNA sequence only in that a ribonucleotide is substituted with a corresponding deoxyribonucleotide in the RNA sequence.

Herein, the meaning of "a new gene was introduced into genome" may mean that some of the entire gene base sequences possessed by an original subject may be substituted with or inserted into a gene derived from a new subject. Due to this introduction, it may become longer or become shorter than a genomic base sequence possessed by the original subject (for example, backbone), or be maintained in length.

"Vaccine strain" used herein is also called a vaccine strain line, and refers to a virus group isolated to be used as a vaccine.

"Attenuated vaccine" used herein is a vaccine made of a weakened respiratory syncytial virus (RSV), and refers to a vaccine having weakened toxicity, as much as a pathogen is still biologically active but it is less likely to cause disease in a host.

"Anti-genome" used herein means a complementary (+)sense polynucleotide molecule acting a template for synthesis of progeny RSV genome.

"Gene encoding a protein" or "protein coding gene" used herein means a gene producing a protein.

"Soluble prefusion F trimeric protein" used herein means a fusion protein in which a linker and a heterogeneous trimerized domain are linked to a soluble F protein ectodomain (preF ectodomain), and in particular, a D486L/E487L/F488W mutation is introduced to a wild type F protein ectodomain to stabilize a protein. The heterogenous trimerized domain may comprise a commonly used foldon domain. The linker is not particularly limited.

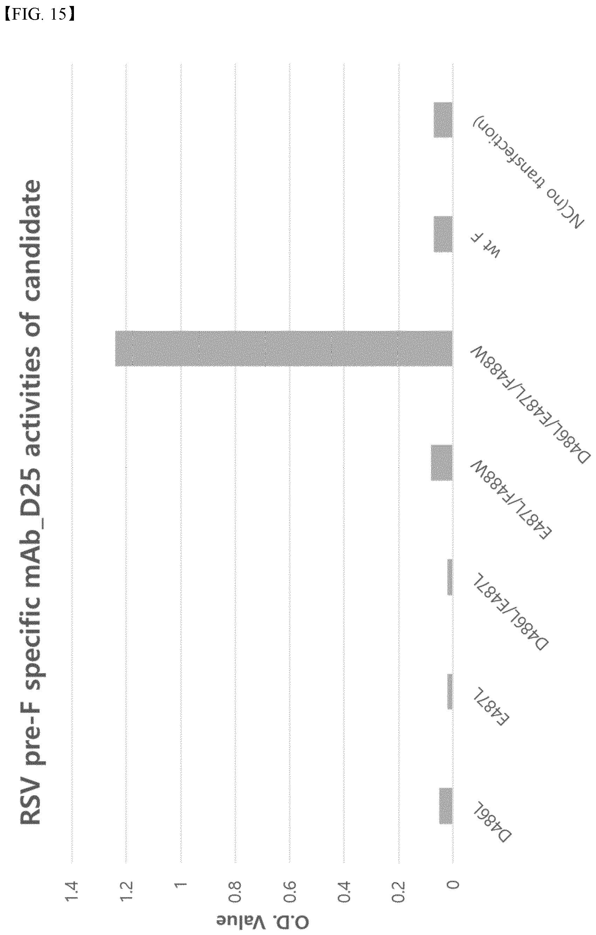

On the other hand, the D486L/E487L/F488W mutation introduced into a wild type F protein was in accordance with the previous research results of the present inventors, and through the following experiment, protein stabilization was confirmed. An RSV F gene comprising a prefusion stabilizing mutation is cloned to a mammalian cell expression vector, and transfected into HEK293FT cells. The following 5 candidates were used as the prefusion stabilizing mutation: D486L, E487L, D486L/E487L, E487L/F488W, and D486L/E487L/F488W. After 5 days, the cells are lysed and reacted on a nickel coated plate to combine an expressed RSV F protein. The combined RSV F protein is combined with an antibody (D25) specifically reacting to a prefusion F protein. After adding a secondary antibody with HRP while binding to the D25 antibody, it is developed with TMB solution and the absorbance is measured at a wavelength of 450nm with a microplate reader. As a result, as shown in FIG. 15, it was confirmed that the RSV F protein comprising the D486L/E487L/F488W mutation binds to the prefusion-specific antibody (D25) among the 5 candidate mutations, and it was confirmed that the F protein was expressed in a prefusion form due to the D486L/E487L/F488W mutation. Therefore, it was confirmed that a mutant virus expressing the prefusion RSV F protein was successfully manufactured.

Hereinafter, an attenuated recombinant RSV with improved safety or stability will be described in detail.

One embodiment provides a recombinant attenuated respiratory syncytial virus comprising a vesicular stomatitis Indiana virus (VSV) G protein (preferably, a protein consisting of the amino acid sequence represented by SEQ ID NO: 2) or an analogue, variant or fragment thereof, or an F protein in which at least one is mutated among furin cleavage sites of an F protein of an RSV. Specifically, for example, in the recombinant attenuated respiratory syncytial virus provided herein, based on RSV genome, a surface glycoprotein of a heterogenous virus is inserted, or an F protein or G protein of an RSV and a surface glycoprotein of a heterogeneous virus are substituted, and thereby, a live RSV with excellent stability and safety is to be provided.

Herein, the protein consisting of the amino acid sequence represented by SEQ ID NO: 2 may be understood to represent a surface glycoprotein G of a recombinant vesicular stomatitis Indiana virus (VSV) (hereinafter, represented by rVSV G protein). This may be encoded by SEQ ID NO: 3 or a polynucleotide sequence having at least 70% sequence homology. The cytoplasmic tail (CT) domain of the VSV G protein may be derived from an RSV F protein. Preferably, the recombinant RSV may comprise not only the protein consisting of the amino acid sequence represented by SEQ ID NO: 2, but also an analogue, variant or fragment thereof, and may comprise even a functional fragment. Herein, `functional fragment' may be understood to comprise a protein having sequence homology of at least 80% or more, 85% or more, 90% or more, 95% or more, 98% or more, or 100% thereto, while maintaining a function of the recombinant VSV G protein. For example, the cytoplasmic tail (CT) domain of the recombinant VSV G protein may include a case where the cytoplasmic tail (CT) domain of the original VSV G protein is linked instead of the RSV F protein.

The VSV is a virus safe to humans, and is similar to the RSV in view of virus classification. Both the VSV and RSV have (-)ssRNA as a genetic material, and are enveloped viruses. In particular, the VSV G protein is a membrane protein which plays a role similar to the RSV F protein, and is a protein having activity alone (not requiring help of other protein), and has stable properties under conditions such as temperature, pH and the like, and thus, it can be confirmed that a safe vaccine with excellent stability can be produced using this.

One embodiment may be provided in a form in which the recombinant attenuated respiratory syncytial virus comprises a protein consisting of the amino acid sequence represented by SEQ ID NO: 2 or a functional fragment thereof, but (or and) further, in the RSV, i) at least one protein selected from the group consisting of SH, G and F proteins is deleted, or ii) at least one protein selected from the group consisting of SH, G and F proteins is substituted, or iii) at least one protein selected from the group consisting of SH, G and F proteins, and proteins other than the deleted protein are substituted with a new protein. Herein, `at least one' may be understood as a meaning including 1 or more, 2 or more, or 3. Herein, `substitution of protein' may include even a case where one or more of an amino acid sequence is changed into other sequence, and may be understood as a broad meaning including replacement of a domain.

In one embodiment, in case of the ii), preferably, it may include substitution of the F protein of the RSV. Preferably, the substitution of the F protein of the RSV may be substituting with a new domain which can reduce infectivity of the RSV. Preferably, the new domain may 1) comprise a fusion protein domain in which a linker and a heterogenous trimerized domain are linked to a soluble F protein ectodomain, or 2) comprise an F protein domain comprising a mutation which increases stability of a prefusion form. Specifically, it may be substituted with a polynucleotide sequence consisting of SEQ ID NOs: 11 or 14, preferably, amino acids encoded by cDNA.

In other embodiment, in case of the iii), together with deletion of the G protein of the RSV, the F protein of the RSV may be substituted with the afore-mentioned new domain which can reduce infectivity of the RSV, and preferably, this may be substituted with a polynucleotide sequence consisting of SEQ ID NO: 12 or SEQ ID NO: 13, preferably, amino acids encoded by cDNA.

Specifically, in the 1) fusion protein domain in which a linker and a heterogenous trimerized domain are linked to a soluble F protein ectodomain, instead of the F protein of the instable RSV, the G protein of the recombinant VSV performs a cell infection role, and the RSV F is inserted in an ectodomain form, so it is not expressed on the cell and virus surfaces, and therefore, it cannot perform the infection role, and instead, when the virus infects cells, it is expressed in cells and secreted to the outside of the cells, and induces only an immune response. In the F ectodomain, a mutation stabilizing into prefusion and a trimerized domain is added, so it may be expressed into a soluble prefusion F trimer. The fusion protein domain in which a linker and a heterogenous trimerized domain are linked to a soluble F protein ectodomain consists of the amino acid sequence represented by SEQ ID NO: 29. The F protein domain comprising a mutation which increases stability of a prefusion form substitutes pre fusion F ectodomain-foldon with single chain F. This consists of the amino acid sequence represented by SEQ ID NO: 31. The single chain F is expressed on the cell and virus surfaces, but is not modified into a postfusion form, so it cannot occur infection, and can perform only an immune inducing role. It is expressed in a state attached to an insoluble cell or virus membrane, so it may induce an immune response much more similar to an actual virus.

In other embodiment, the recombinant attenuated respiratory syncytial virus (RSV) may comprise a mutated F protein having a mutation in at least one of furin cleavage sites possessed by the F protein of the RSV. Preferably, in the mutated F protein, the furin cleavage site I or II of the F protein of the RSV may be modified into other protease cleavage site such as trypsin, MMP, trypsin-like protease and the like, and more preferably, the peptide of SEQ ID NO: 55 corresponding to the furin cleavage site II of the F gene which is the 106~109th amino acid sequence of the F protein may be modified into the peptide of SEQ ID NO: 56, or the peptide of SEQ ID NO: 57 corresponding to the furin cleavage site I of the F gene of the 133~136th amino acids may be substituted with the peptide of SEQ ID NO: 58. The F protein is cleaved after transcription by furin protease rich in ER, and divided into F1/F2 subdomains, and becomes in a metastable state. Preferably, when the furin cleavage site I or II is changed to a cleavage site by cleavage enzyme abundant in lysosome such as trypsin like protease and the like, cleavage does not occur and it may be present on the virus surface in a single chain F form with high stability, and it is effective in inducing an immune response.

In other embodiment, the virus is one in which genes encoding NS1 and NS2 proteins comprised in the virus are further substituted, and the antigenomic cDNA of the substituted genes may consist of the nucleotide sequences represented by SEQ ID NOs: 32 and 33, respectively.

Specifically, a recombinant attenuated RSV with increased safety by deoptimizing codons of polynucleotides encoding the NS1 protein, NS2 protein, or both the NS1 and NS2 proteins for human codons may be provided. Codon deoptimization of the NS1 and/or NS2 proteins can reduce the expression level in the human body of the NS1 or NS2 protein, thereby attenuating the recombinant virus, and increasing safety.

The recombinant RSV may be used safely as a live vaccine strain.

One example of the present invention provides a nucleotide molecule composing a recombinant attenuated RSV, and this comprises genome or anti-genome of the RSV. One embodiment provides a recombinant vector of a recombinant attenuated RSV comprising the same, and the antigenomic cDNA sequence comprised in the vector consists of any one or more nucleotide sequences selected from the group consisting of SEQ ID NOs: 6 to 16.

The recombinant vector comprises a nucleotide sequence encoding the rVSV G protein. Preferably, the nucleotide sequence encoding the rVSV G protein may be positioned between the SH gene and G gene of the RSV, and this may be represented by SEQ ID NO: 3.

The genome of the recombinant attenuated RSV may be provided in a form in which i) a nucleotide sequence encoding at least one protein selected from the group consisting of SH, G and F proteins or a gene encoding the protein is deleted, or ii) at least one protein selected from the group consisting of the SH, G and F proteins is substituted, or iii) a nucleotide sequence encoding at least one protein selected from the group consisting of the SH, G and F proteins or a gene encoding the protein is deleted and proteins other than the deleted protein are substituted with other protein, while comprising nucleotide sequence encoding the rVSV G protein.

In one embodiment, in case of the ii), the nucleotide encoding the F protein of the RSV may be substituted with a nucleotide sequence encoding a soluble preF trimer protein or preFsc protein. Preferably, the cDNA sequence of the recombinant RSV may comprise a nucleotide consisting of SEQ ID NO: 11 or SEQ ID NO: 14, respectively, or a functional fragment thereof.

In other embodiment, in case of the iii), the nucleotide encoding the F protein of the RSV in which the nucleotide or gene encoding the G protein of the RSV is deleted may be substituted with a nucleotide sequence encoding a soluble preF trimer protein or preFsc protein. Preferably, the cDNA sequence of the recombinant RSV may comprise a nucleotide preferably consisting of SEQ ID NO: 12 or SEQ ID NO: 13, respectively, or a functional fragment thereof.

In one embodiment, the genome of the recombinant attenuated respiratory syncytial virus (RSV) may have a mutation in at least one of the proteins encoding the furin cleavage sites of the F protein. In the nucleotide sequence encoding the mutated F protein, SEQ ID NO: 50, which is the nucleotide sequence corresponding to furin cleavage site II of the F gene positioned from the 6137th to 6148th of the nucleotide sequence encoding the F protein may be modified into SEQ ID NO: 51, or SEQ ID NO: 52, which is the nucleotide sequence corresponding to furin cleavage site I of the F gene positioned from the 6218th to 6229th may be substituted with SEQ ID NO: 53.

In other embodiment, the genes encoding the NS1 and NS2 proteins of the virus may be provided by being substituted with genes consisting of the nucleotide sequence represented by SEQ ID NOs: 32 and 33, respectively.

It is the fact already known in the art that it is present as the gene arrangement order of the RSV is arranged as 3'leader-NS1, NS2, N, P, M, SH, G, F, M2, L-5' trailer.

In a specific example, the nucleotide sequence of the recombinant RSV may comprise any one nucleotide sequence selected from the group consisting of SEQ ID NOs: 6 to 16. The nucleotide sequence of the recombinant RSV may be inserted into a vector for co-transfection, and in this case, it may be present with a T7 promoter, a hammerhead ribozyme, a hepatitis delta virus ribozyme, and a T7 terminator required for producing a recombinant virus, and preferably, it may be presented by being inserted as any one nucleotide sequence selected from the group consisting of SEQ ID NOs: 17 to 27.

Preferably, the RSV virus genome, which is the basis of the present invention, may use the human RSV A virus strain of SEQ ID NO: 1. The RSV virus strain are various including RSV A strain, RSV B strain, HRSV A strain, HRSV B strain, BRSV strain, avian RSV strain and the like, but on the purpose of the present invention, the RSV A virus strain may be preferable as the RSV basic backbone in which a foreign gene is inserted.

One example of the present invention provides an isolated polynucleotide molecule comprising a nucleic acid encoding an F protein of a stabilized pre-fusion respiratory syncytial virus (RSV) or an analogue, variant or fragment thereof; or a nucleic acid encoding a G protein of vesicular stomatitis Indiana virus (VSV) or an analogue, variant or fragment thereof. The nucleic acid is antigenomic cDNA or RNA, and preferably, the polynucleotide molecule comprises antigenomic cDNA of the recombinant attenuated RSV genome, and the cDNA comprises a polynucleotide encoding antigenome of the recombinant attenuated RSV. In one embodiment, the isolated polynucleotide molecule may provide a polynucleotide molecule which is cDNA consisting of a polynucleotide represented by any one or more sequences selected from the group consisting of SEQ ID NOs: 6 to 16.

One embodiment provides a vector comprising the isolated polynucleotide molecule, preferably, the antigenomic cDNA. One embodiment provides a cell comprising an isolated polynucleotide molecule or vector.

The polynucleotide may be comprised in a vector or expressed by a vector to produce a recombinant RSV. Therefore, the cell transfected with the isolated polynucleotide or vector falls within the scope of the present invention is also exemplified herein. In a related aspect of the present invention, a composition (e.g., isolated polynucleotide and vector including RSV-encoding cDNA) and a method for producing a recombinant RSV are also provided. In addition, the same or a different expression vector comprising at least one isolated polynucleotide molecule encoding the RSV protein is provided. This protein may be also directly expressed from the genome or antigenomic cDNA. The vector(s) may be preferably expressed or co-expressed in cells or cultures in which cells are cultured to produce a recombinant attenuated RSV.

One embodiment provides a method for producing a recombinant attenuated RSV comprising the followings. It may comprise S1) transfecting the vector with a host cell, S2) culturing the cell or its culture for a sufficient time to allow virus replication, and S3) isolating the replicated recombinant RSV.

Specifically, in the S1) transfecting, at least one of the vectors may be used, and preferably, two or more may be used. Specifically, the vector may comprise a first expression vector comprising the recombinant antigenomic cDNA and a second expression vector comprising a polynucleotide encoding any one or more proteins selected from the group consisting of N, P, L, and M2-1 proteins. The RSV is a negative sense RNA virus, and a separate helper gene required for gene synthesis is needed, when a virus is produced in vitro using reverse genetics. The recombinant RSV antigenome and polynucleotide encoding each of the N, P, L, and M2-1 proteins may be introduced into cells by a method such as transfection, electroporation, mechanic insertion and introduction, and all cell infection methods of a plasmid commonly used in the art may be used. Preferably, as the cell, a cell such as a vero cell and the like may be used, and it may be advantageous to produce the recombinant RSV virus of the present invention in the vero cell. The method for introducing the recombinant RSV antigenomic cDNA and polynucleotide encoding each of the N, P, L, and M2-1 proteins may use a method commonly used in the art, but not limited thereto. In another embodiment, it may be synthesized by synthesizing the RSV antigenomic RNA extracellularly and transfecting into a cell expressing RSV proteins. Preferably, the vector comprising the recombinant RSV antigenome may be a cloning vector comprising cDNA of a polynucleotide comprising a T7 promoter, a hammerhead ribozyme, RSV antigenome (or antigenomic cDNA), a hepatitis delta virus ribozyme, and a T7 terminator.

The polynucleotide encoding the recombinant RSV antigenome may be comprised in the first expression vector, and the helper gene may be comprised in a vector different from the first expression vector (second expression vector), or may be comprised in the same vector.

The vector comprising the polynucleotide encoding the recombinant RSV antigenome or antigenomic cDNA may exemplarily use pCC1 plasmid, and may stabilize the recombinant RSV antigenome, and as long as it does not inhibit the purpose of the present invention, the type of the vector may be used without limitation. The vector comprising the polynucleotide encoding the recombinant RSV antigenome or antigenomic cDNA may modify the vector by inducing mutation, modifying a restriction enzyme site, or inserting a synthesized polylinker comprising a controlled restriction enzyme site on the purpose of facilitating combination of genes.

One embodiment provides a recombinant attenuated RSV produced by the method.

In one aspect, the present invention comprises a method for producing an RSV comprising infecting a host cell with a recombinant RSV to which RSV infection is allowed under a condition of allowing RSV proliferation in the infected cell. After the period of replication in a culture, the cell is dissolved and a recombinant RSV is isolated therefrom. If necessary, at least one desired RSV is isolated to produce at least one RSV for vaccine, diagnosis and other uses.

One embodiment provides a respiratory syncytial virus immunogenic composition comprising a recombinant attenuated RSV.

The immunogenic composition prepared by one embodiment of the present invention may have both safety and immunogenicity, and therefore, a composition which performs an immunity inducing action by preparing infection of a respiratory syncytial virus and has high safety by introducing the recombinant respiratory syncytial virus according to one example in the body. The recombinant attenuated RSV prepared by one embodiment of the present invention may be used as a vaccine strain, preferably, a live vaccine strain, for preventing RSV infection, and when the recombinant RSV is used for vaccine and/or immunogenic composition uses, the virus prepared according to the present invention may be directly used as a vaccine preparation, or used by being freeze-dried, or used by being mixed in liquid. The vaccine and/or immunogenic composition of the present invention comprises any pharmaceutical substance which does not cause a harmful immune response to a vertebrate administered with this composition by itself, and comprises a pharmaceutically acceptable carrier comprising any appropriate diluent or excipient which can be administered together with the recombinant RSV vaccine strain without excessive toxicity. In addition, if needed, a pharmaceutically acceptable vaccine protecting agent, immunopotentiator, diluent, absorption enhancing agent or the like may be comprised. The vaccine protecting agent includes for example, a mixture of lactose phosphate and glutamate gelatin, but not limited thereto. The vaccine may contain propylene glycol and a sufficient amount (e.g.: about 1 %) of sodium chloride for preventing hemolysis, if needed, in case of liquids or injections. The term "pharmaceutically acceptable" means what is listed in the United States Pharmacopoeia, European Pharmacopoeia, or other generally recognized pharmacopoeia for use in vertebrates and more specifically, humans. The RSV live vaccine strain of the present invention is administered in an effective dose or amount (defined above) sufficient for stimulating an immune response against at least one strain of RSV viruses. This composition may be used as a vaccine and/or an immunogenic composition for inducing a protective immune response in vertebrates.

In the present invention, the vaccine and/or immunogenic composition may further comprise an adjuvant. "Adjuvant" means any substance added to a vaccine composition to enhance antigenicity of an RSV virus. The substance used as the adjuvant is not particularly limited, and for example, it may be aluminum hydroxide, MPL (monophosphoryl lipid A), or oil or a supplementary molecule which is added to a vaccine or generated by the body after each induction with this additional component. In the present invention, the vaccine composition may induce cytokine production against an RSV, and the cytokine may be at least one selected from the group consisting of interferon-gamma (hereinafter, IFN-γ), interleukin-12 (hereinafter, IL-12), and tumor necrosis factor-alpha (hereinafter, TNF-α), but not limited thereto.

In one example of the present invention, the vaccine and/or immunogenic composition may induce production of an antibody against an RSV and/or vesicular stomatitis Indiana virus (VSV). The vaccine and/or immunogenic composition may induce production of a neutralizing antibody against the RSV and/or VSV.

The vaccine or immunogenic composition of the present invention may be administered into a subject to induce an immune response against an RSV. In one embodiment, the subject refers to a subject in need of preventing RSV infection, and specifically, includes animals, and the animals may include mammals such as humans, non-human primates, mice, rats, cats, dogs, horses, and the like. In common, the dosage may be adjusted in this range based on for example, age, body condition, body weight, food, administration time and other clinical factors. The present inventor comprises a method for formulating a vaccine or immunogenic composition by adding an effective dose of immunogen into the preparation to causing substantial immunity against infection of a subject or its at least one symptom. Stimulation of substantial immunity by a single dosage is preferably, but in order to obtain a desired effect, an additional dosage may be administered through the same or another route. In neonates and infants, for example, to induce immunity at a sufficient level, multiple administration may be needed. In one non-restrictive embodiment, the dosage of the recombinant RSV live vaccine strain comprised in a vaccine is in a range of commonly, about 3.0 log₁₀ to about 6.0 log₁₀ plaque forming units ("PFU") or more viruses, more generally, about 4.0 log₁₀ to 5.0 log₁₀ PFU viruses, per patient. In one embodiment, about 5.0 log₁₀ to 6.0 log₁₀ PFU per patient may be administered, for example, during infancy between 1 to 6 months after birth, and the dosage of at least once additional booster may be administered for 2 to 6 months after the first dosage or more. In another embodiment, young infants may be administered at a dosage of about 5.0 log₁₀ to 6.0 log₁₀ PFU at about 2, 4, 6 months. In order to maintain protection at a sufficient level against infection, if needed, it may continue at intervals throughout childhood. In one embodiment, a method for inducing substantial immunity for virus infection or its at least one symptom in a subject comprises administering an effective dose of RSV recombinant virus at least once.

The method for administering a vaccine and/or an immunogenic preparation includes parenteral administration (for example, intramuscular, intravenous, intradermal and subcutaneous), epidural and mucosal (for example, nasal and oral or pulmonary route or suppository), but not limited thereto. In a specific embodiment, the composition is administered through an intranasal, intratracheal, intramuscular, intradermal or subcutaneous route. The composition may be administered through any convenient route by absorption through for example, injection or transient injection, or the inside of epithelium or mucosa (for example, oral mucosa, colon, conjunctiva, nasopharynx, central pharynx, vagina, urinary tract, bladder, intestinal mucosa, etc.), and may be administered with other biologically active substance. The administration may be systemic or local. A preventive vaccine preparation is administered systemically by a subcutaneous or intramuscular injection using a needle and a syringe or a needleless injection device. Selectively, the vaccine preparation is administered nasally by droplets, large particle aerosol (larger than about 10 microns) or spray into the upper respiratory tract. While any of the routes of delivery causes an immune response, the nasal administration provides an increased effect by causing mucosal immunity at a position of virus penetration. In another embodiment, the vaccine and/or immunogenic preparation may be administered by a method for targeting mucosal tissue to induce an immune response at a site of immunization. The administration site is not limited.

One embodiment provides a pharmaceutical composition for prevention or treatment of a respiratory syncytial virus comprising the immunogenic composition.

One embodiment of the present invention provides a method for preventing infection of a respiratory syncytial virus (RSV) by administering the RSV vaccine strain of the present application into a subject.

One embodiment provides a method for inducing an immune response against an RSV in a subject, comprising administering an effective amount of the pharmaceutical composition which produces immune into a subject.

One embodiment provides a use of a recombinant RSV, for inducing an immune response against an RSV in a subject.

One embodiment provides a recombinant attenuated RSV used as a live vaccine strain for preventing RSV infection. The viruses of the present invention can reduce at least one functional characteristic of the viruses by being attenuated. In a specific example, attenuation may be measured by comparing with the wild type virus strain from which the attenuated virus is derived. In another example, attenuation may be determined by comprising with the growth of the attenuated virus in other host system. The recombinant viruses of the present invention may be further designed to show genetically attenuated phenotypes. The recombinant viruses of the present invention show attenuated phenotypes so that the viruses can be administered as a vaccine. The attenuation may be achieved with any method known to those skilled in the art. Without being limited to any theory, the recombinant virus can be constructed with attenuated phenotypes.

The protein described in the present description may be understood to include peptides or polypeptides as a set of amino acid sequences, and may be interchangeably used.

The nucleotide used in the present description may be used as a meaning including polynucleotides, and may be interchangeably used.

The nucleotide used in the present description may be understood to include functional fragments thereof. For example, if a desired function or effect is shown in a sequence with sequence homology of 85% or more, 90% or more, 95% or more, 99% or more, or 100%, it may be understood that the right of the present invention extends to a case where it has the sequence homology.

The protein used in the present description may be understood to comprise its functional fragment. For example, when a desired function or effect is shown in a sequence with sequence homology of 85% or more, 90% or more, 95% or more, 99% or more, or 100%, it can be understood that the right of the present invention extends to a case where it has the sequence homology.

Hereinafter, in order to help understanding of the present invention, it will be described in detail by examples and the like. However, the examples according to the present invention may be modified into various other forms, and the scope of the present invention should not be construed as limited to the following examples. The examples of the present invention are provided to describe the present invention more completely to those with average knowledge in the art to which the present invention pertains.

### 1. Preparation of wild type RSV A virus strain

A wild type RSV A virus strain having the following information was prepared as follows.
- Definition ; Human respiratory syncytial virus strain RSVA / TH_10654 / complete genome
- Accession No. ; KU950464.1
- Length ; 15232 bp
- Host ; Homo sapiens / female / 12 weeks
- Collection date ; 19-Feb-2014
- Country ; USA
- Subtype ; RSV A(RSV A2 strain)

### 2. Selection of surface glycoprotein donor virus

As a surface glycoprotein donor, a vesicular stomatitis Indiana virus (VSV) was selected.

### 3. Preparation of cDNA encoding RSV antigenome

It was manufactured based on the following backbone construct A.

### (1) Design of backbone construct A

The gene order is the order of 5'- T7 promoter - hammerhead ribozyme - RSV anti-genome (mutation portion) - hepatitis delta virus ribozyme - T7 terminator -3'. The mutation portion is based on SEQ ID NO: 1.

A T7 promoter sequence (SEQ ID NO: 38) was inserted into the 5' end. Following the T7 promoter sequence, a hammerhead ribozyme sequence (SEQ ID NO: 39) was inserted. Following the hammerhead ribozyme sequence, a wild type RSV anti-genome sequence was inserted and the following mutation was applied. In other words, the mutation portion was manufactured to insert any one cDNA sequence selected from the group consisting of SEQ ID NOs: 6-16, instead of SEQ ID NO: 1.

**The process of manufacturing cDNA in which the mutation portion is introduced is as follows:**
Based on the anti-genome sequence of the wild type RSV,
GCGCGCC is inserted between 77nt and 78nt to produce an AscI restriction enzyme sequence. (As the AscI restriction enzyme sequence is GGCGCGCC<8 bp>, but the sequence of 77nt is G, so only GCGCGCC<7 bp> is inserted)
   1) Insert the CCTGCAGG (SbfI restriction enzyme) sequence between 1079nt and 1080nt.
   2) Insert the GGCCGGCC (FseI restriction enzyme) sequence between 4590nt and 4591nt.
   3) Substitute ATG(M) that is 4799nt and 4802nt bases with the ATT(I) sequence.
   4) Insert the ACGCGT (MluI restriction enzyme) sequence between 5639nt and 5640nt.
   5) Insert the GGGCCC (ApaI restriction enzyme) sequence between 7595nt and 7596nt.
   6) Insert the hepatitis delta virus ribozyme sequence (SEQ ID NO: 40) following the wild type RSV antigenome sequence.
   7) Insert the T7 terminator sequence (SEQ ID NO: 41) following the hepatitis delta virus ribozyme sequence.
   8) Insert the sequence of SEQ ID NO: 42 to the 5' end, and insert the sequence of SEQ ID NO: 43 to the 3' end. Herein, SEQ ID NO: 42 inserted to the 5' end was indicated as a 3' leader, and SEQ ID NO: 43 inserted to the 3' end was indicated as a 5' trailer.

After synthesizing designed recombinant cDNA (SEQ ID NO: 1) in vitro, it was cloned in front of a chloramphenicol-resistant gene of a pCC1 cloning vector. Herein, one point mutation induction (M48I) of the G protein sequence was introduced.

Hereinafter, various mutations were introduced into the RSV anti-genome of the cDNA. The process of making the mutation portion was specifically shown below.

### (2) Example 1. cDNA construct (SEQ ID NO: 6) of modified RSV with chimeric vesicular stomatitis virus (VSV) G inserted

The VSV G gene sequence was inserted between the SH gene and G gene of the backbone construct A of SEQ ID NO: 1. As the VSV G sequence, the G sequence of GenBank FJ478454.1, which is the Indiana serotype used as a viral vector. Then, as the cytoplasmic tail region of the VSV G (SEQ ID NO: 44), the chimeric VSV G sequence replaced with the cytoplasmic tail region of the wild type human respiratory syncytial virus strain RSVA/TH_10654 (SEQ ID NO: 45) was used. For 5' UTR and 3' UTR, the UTR sequence of the G protein gene of the wild type human respiratory syncytial virus strain RSVA/TH_10654 was used, and in front of 5' UTR, the NS1/NS2 inter-gene was inserted (SEQ ID NO: 3). Backbone construct A (SEQ ID NO: 1) was cut with FseI restriction enzyme and the recombinant chimeric VSV G gene (SEQ ID NO: 3) was inserted.

### (3) Example 2. Removal SH from construct (SEQ ID NO: 6) in which chimeric VSV G is inserted (SEQ ID NO: 7)

The SH gene was removed to further attenuate the construct of SEQ ID NO: 6. From the SEQ ID NO: 6, the 3'UTR-FseI part of SH from the 3'UTR of M (4288 ~ 4712) was removed, and an RsrII restriction site (CGGTCCG) site was added.

### (4) Example 3. Removal of G gene from SEQ ID NO: 7 (SEQ ID NO: 8)

SEQ ID NO: 7 was cut with FseI, MluI to remove the part comprising the G gene (5896-6934), and the inter-gene (SEQ ID NO: 46) was inserted between the G/F genes of the wild type human respiratory syncytial virus strain RSVA / TH_10654 (SEQ ID NO: 8).

### (5) Example 4. Removal of F gene from SEQ ID NO: 7 (SEQ ID NO: 9)

SEQ ID NO: 7 was cut with MluI, ApaI to remove the part comprising the F gene (6941-8896) and the inter-gene (SEQ ID NO: 46) was inserted between the G/F genes of the wild type human respiratory syncytial virus strain RSVA / TH_10654 (SEQ ID NO: 9).

### (6) Example 5. Replacement of Fe gene of backbone construct A with VSV G gene (SEQ ID NO: 10).

SEQ ID NO: 1 was cut with MluI, ApaI to remove the 5758-7713 part and the recombinant chimeric VSV G (SEQ ID NO: 3) was inserted into this position.

### (7) G/F inter-gene - F 5' UTR - preF ectodomain-foldon fusion gene - F 3'UTR (SEQ ID NO: 4).

G/F inter-gene of the wild type human respiratory syncytial virus strain RSVA / TH_10654, F 5' UTR, a variant gene in which a mutation was applied to the ectodomain (1~513 aa) of the F protein by D486L, E487L, F488W, a linker (SEQ ID NO: 54), T4 fibritin foldon gene, and F 3' UTR were synthesized (SEQ ID NO: 4).

### (8) Example 6. SEQ ID NO: 10 was cut with ApaI, and SEQ ID NO: 4 was inserted to the part (SEQ ID NO:11).

### (9) Example 7. Removal of G gene from SEQ ID NO: 11 (SEQ ID NO: 12).

SEQ ID NO: 11 was cut with FseI, MluI to remove the 4713-5751 part comprising the G gene and the SH/G inter-gene (SEQ ID NO: 47) was inserted (SEQ ID NO: 12).

(10) The F protein is separated into 2 subunits of F1 and F2 and a peptide consisting of 27 amino acids between them (p27) as cleavage occurs at two locations by furin protease. After cleavage, F1 and F2 are linked by 2 disulfide bonds, but p27 is removed from F and becomes a prefusion form. The hydrophobic fusion peptide (FP) region positioned at the N terminus of the F1 in the prefusion form is located inside the protein, but it is in a thermodynamically very instable state, and therefore, it easily comes out from the inside and is exposed to the outside, and it is irreversibly transformed into a postfusion form. When the furin cleavage site is mutated to prevent cleavage and the P27 part is removed, the FP part located inside the protein cannot protrude to the outside, and therefore, it is stabilized in a prefusion form. When the protein stabilized in this way is present on the virus surface, it cannot perform a cell fusion function, so the infectivity of the virus disappears, but when a heterogenous attachment/fusion protein such as VSV G is present together on the virus surface, while maintaining the infectivity, prefusion F-specific antibodies can be induced.

To the WT F of the wild type human respiratory syncytial virus strain RSVA / TH_10654, 1) R106G mutation is introduced, and 2) 29 amino acids (SEQ ID NO: 48) corresponding to R109~F137 comprising 2 furin cleavage sites and p27 region are removed, and 3) the G/F inter-gene and F 5' UTR are linked to 5' of the DNA sequence encoding a modified F protein in which the part in which 29 amino acids are removed is linked with the peptide of SEQ ID NO: 49, and F 3'UTR is linked to 3' (SEQ ID NO: 5).

### (11) Example 8. The sequence that SEQ ID NO: 12 was cut with ApaI to remove the 6361-8229 part and SEQ ID NO: 5 is inserted is SEQ ID NO: 13.

(12) Example 9. SEQ ID NO: 11 was cut with AscI, SbfI to replace the 174-1175 part comprising NS1, NS2 genes with NS1 and NS2 coding parts deoptimized for human codons (SEQ ID NO: 14). NS1 and NS2 genes are genes involved in immune evasion, and when deoptimized for human codons, the expression level is reduced in human cells and the virus growth is deteriorated and it is attenuated for a human body and the safety increases, but it is not attenuated in production cells lacking this immune mechanism, so the production does not decrease.

(13) Example 10. Furin cleavage site II (6137~6148, SEQ ID NO: 50) of the F gene of backbone construct A (SEQ ID NO: 1) was mutated with SEQ ID NO: 51 (SEQ ID NO: 15). The F protein is cut after transcription by furin protease rich in ER and divided into F1/F2 subdomains, and becomes a metastable state. When the furin cleavage site is changed to a cleavage site of cleavage enzyme abundant in lysosome, it is expected that cleavage will not occur after transcription and it is present in a single chain F form with high stability on the virus surface.

(14) Example 11. Furin cleavage site I (6218~6229, SEQ ID NO: 52) of the F gene of backbone construct A (SEQ ID NO: 1) was mutated with SEQ ID NO: 53 (SEQ ID NO: 16). When the furin cleavage site is changed to a cleavage site of cleavage enzyme abundant in lysosome, it is expected that cleavage will not occur after transcription and it is present in a single chain F form with high stability on the virus surface.

The cDNA sequences of SEQ ID NOs: 17-27 were introduced into the vectors for cDNA co-transfection into which the examples were introduced

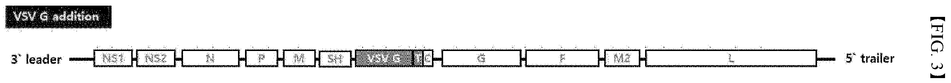

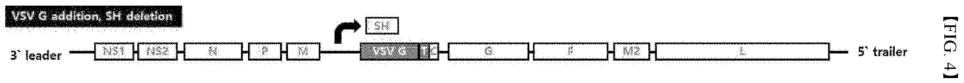

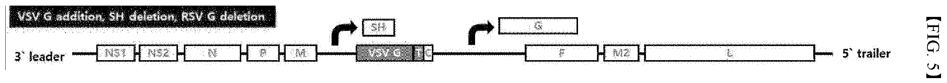

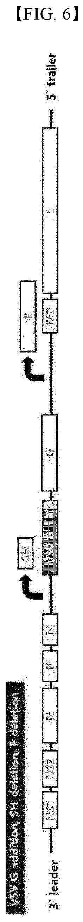

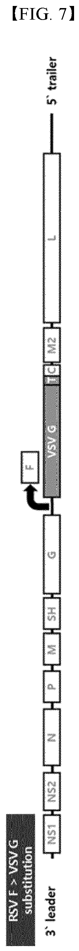

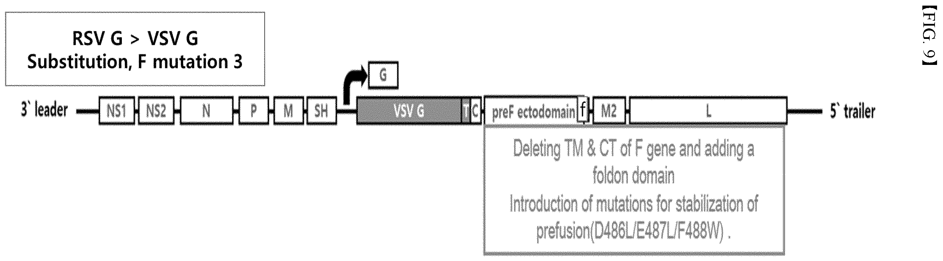

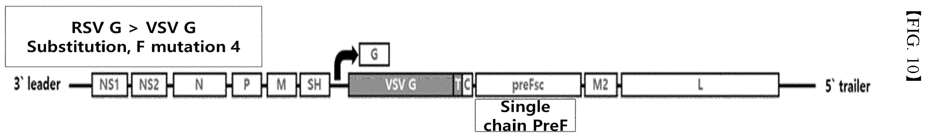

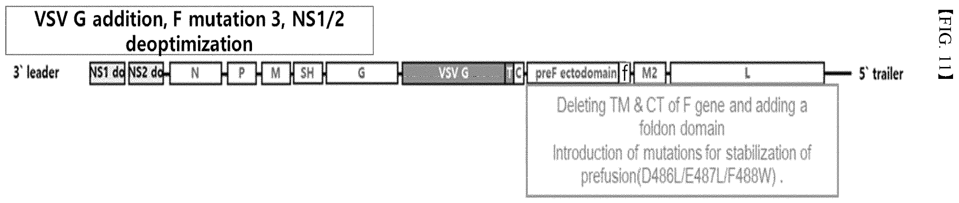

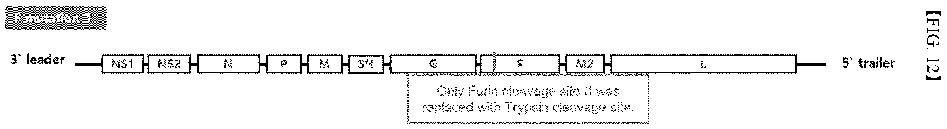

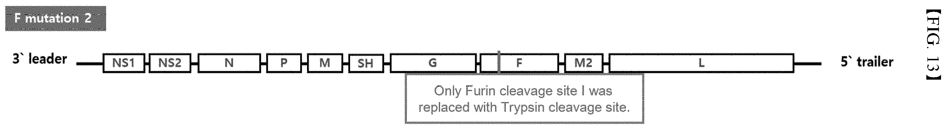

The recombinant RSV vaccine strain candidates designed in each example were summarized below. Hereinafter, Δ is understood as deletion. On the right, the name of the cDNA vector for producing a mutant virus was written. Schematic diagrams of negative strand RNA (i.e., viral RNA) of each cDNA were shown in FIGs. 3 to 13, respectively.
1) RSV backbone strain ; wtRSVA_TH10654(Wild type control)
2) RSV backbone strain- VSV G insertion ; cRSVA_VSVG_A (FIG. 3)
3) RSV backbone strain- VSV G insertion, SH deletion ; cRSVA_VSVG_A_ΔSH (FIG. 4)
4) RSV backbone strain- VSV G insertion, SH deletion, RSV G deletion ; cRSVA_VSVG_A_ΔSH_ΔG (FIG. 5)
5) RSV backbone strain- VSV G insertion, SH deletion, F deletion ; cRSVA_VSVG_A_ΔSH_ΔF (FIG. 6)
6) RSV backbone strain- VSV G substitution ; cRSVA_VSVG_S (FIG. 7)
7) RSV backbone strain- VSV G insertion, preF ectodomain-foldon mutation ; cRSVA_VSVG_A_preF_ef (FIG. 8)
8) RSV backbone strain- VSV G substitution, preF ectodomain-foldon mutation ; cRSVA_VSVG_S_preF_ef (FIG. 9)
9) RSV backbone strain- VSV G substitution, preF single chain mutation ; cRSVA_VSVG_S_preF_sc (FIG. 10)
10) RSV backbone strain- VSV G insertion, preF ectodomain-foldon mutation, NS1/NS2 deoptimization ; cRSVA_VSVG_A_preF_ef_NS1/NS2deop (FIG. 11)
11) RSV backbone strain- F furin cleavage site II mutation ; cRSVA_ mF1 (FIG. 12)
12) RSV backbone strain- F furin cleavage site I mutation ; cRSVA_ mF2 (FIG. 13)

### 4. Design of helper genes (4 kinds)

The RSV is a negative sense RNA virus, and when producing the virus in vitro, helper gene required for gene synthesis (N, P, L, M2-1 protein encoding genes of the RSV) are added together.
(1) The N protein gene is cloned into pCI neo vector using restriction enzymes XhoI and MluI with the 1119 ~ 2294 sequence of the wild type human respiratory syncytial virus strain RSVA / TH_10654 anti-genome.
(2) The P protein gene is cloned into pCI neo vector using restriction enzymes XhoI and MluI with the 2326 ~ 3051 sequence of the wild type human respiratory syncytial virus strain RSVA / TH_10654 anti-genome.
(3) The M2-1 protein gene is cloned into pCI neo vector using restriction enzymes XhoI and MluI with the 7647 ~ 8231 sequence of the wild type human respiratory syncytial virus strain RSVA / TH_10654 anti-genome.
(4) The L protein gene is cloned into pCI neo vector using restriction enzymes XhoI and MluI with the 8539 ~ 15036 sequence of the wild type human respiratory syncytial virus strain RSVA / TH_10654 anti-genome.

The N protein gene, the P protein gene, the M2-1 protein gene, and the L protein gene were represented by SEQ ID NO: 34, SEQ ID NO: 35, SEQ ID NO: 36 and SEQ ID NO: 37, respectively.

### 5. Virus rescue

BHK cells were prepared in a 6well plate at a concentration of 5 × 10⁵ cell/well.

Next day, 0.5ug each of a total of 6 kinds of plasmids of the T7 polymerase expression vector, RSV full length anti-genome vector, and 4 kinds of helper gene vectors (N, P, M2-1, L) was simultaneously transfected into the BHK cells using lipofectamine3000.

After 10 days, the culture solution was recovered, and the virus was detected by immunofluorescence assay (IFA). Specifically, the transfected BHK cells were fixed with 4% PFA at a room temperature for 30 minutes, and the cell permeability increased using Triton X100, and immunofluorescence was performed using the primary antibodies, Mouse anti-RSV N mAb, Mouse anti-RSV G mAb, Mouse anti-RSV F mAb, Mouse anti-VSV G mAb, and secondary antibodies in which Rhodamine Green (Invitrogen) was combined to the primary antibodies. The nuclei of the cells were stained using Hoechst 33342. Fluorescent signals were confirmed by visualizing them with a fluorescent microscope.

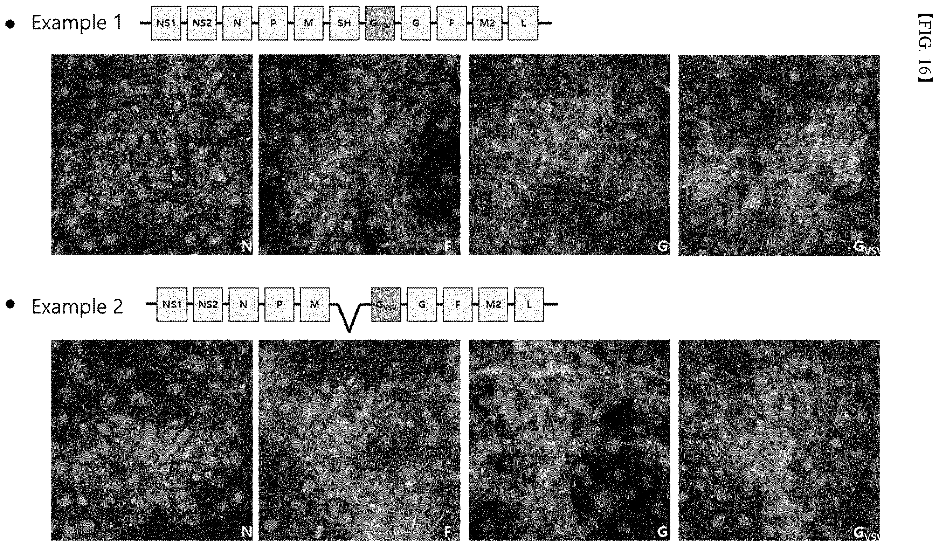

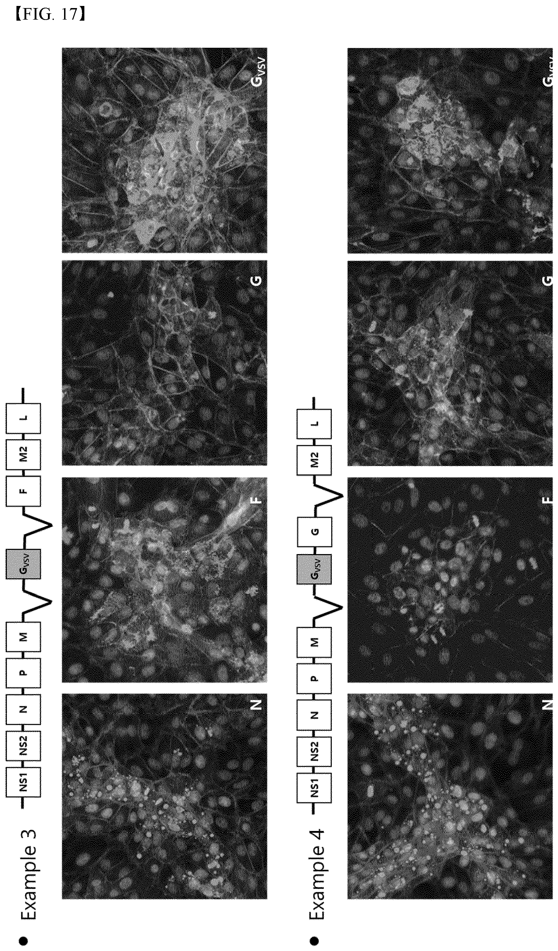

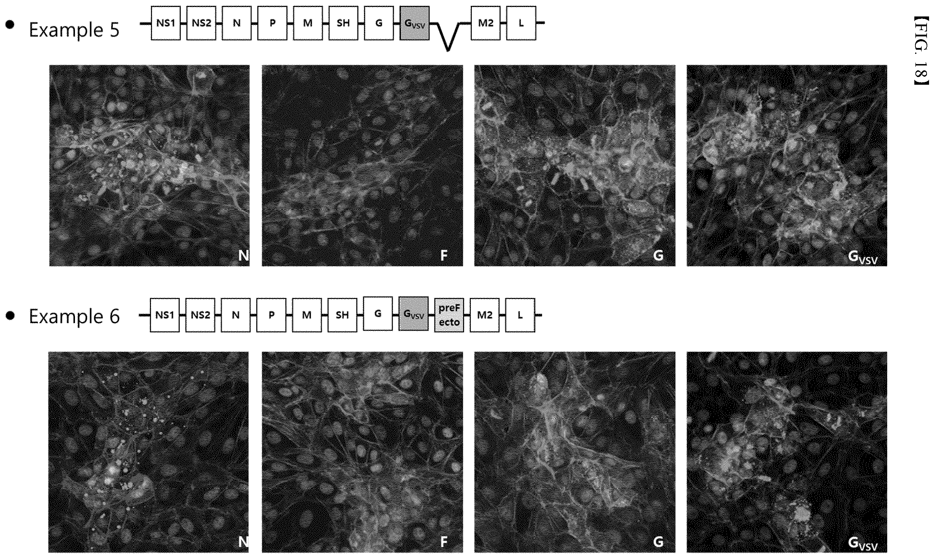

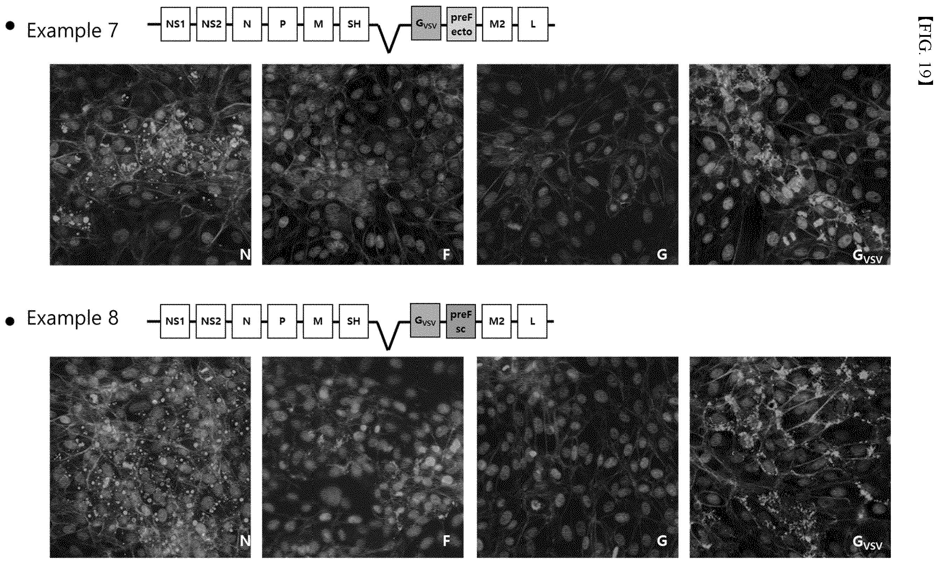

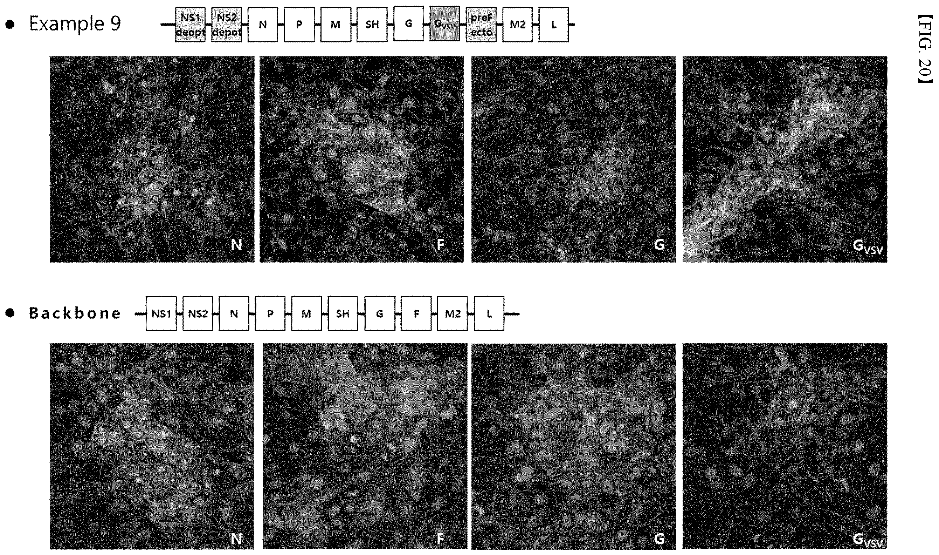

As a result, as could be confirmed in FIGs. 16 to 20, it was confirmed that the designed recombinant viruses were rescued well.

### 6. In vitro attenuation test

0.1 MOI of the 11 kinds of viruses including the backbone strain was infected into Vero cells, HEp2 cells, MRC-5, BEAS-2B, NHBE (primary normal human bronchial epithelial cells) or HAE (primary human tracheobronchial airway cells), and they were cultured for 7 days. At an interval of every 1 day, the virus culture solution was collected, and the virus titer was analyzed using q-PCR or plaque assay, and it was confirmed that the mutant viruses, the vaccine strains were attenuated as the proliferation rate and proliferation titer were reduced, compared to the wild type virus.

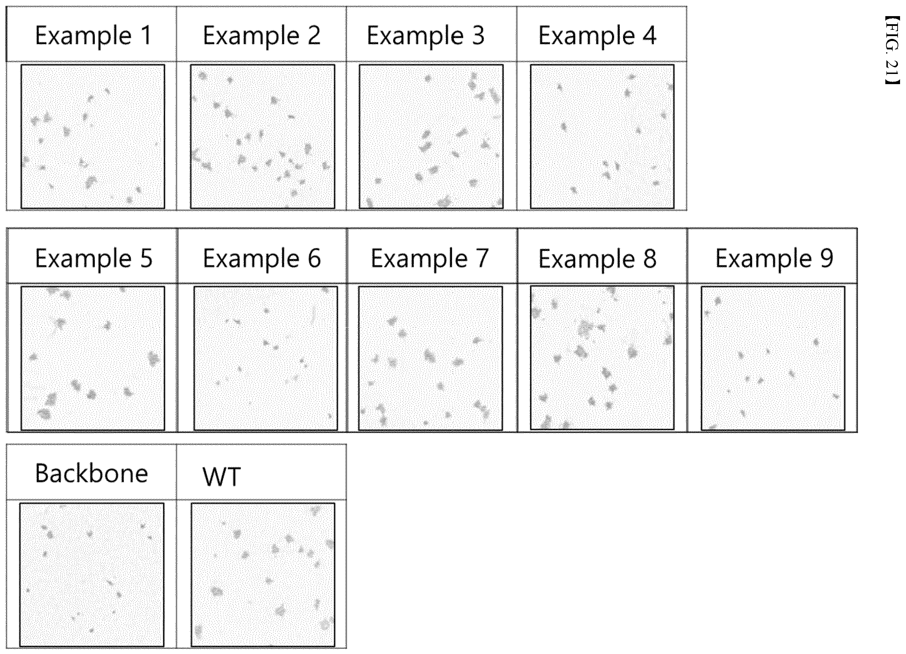

In addition, through plaque assay, by comparing the pattern of the plaque with naked eyes, whether attenuation progressed was confirmed. As a result, as could be confirmed in FIG. 21, it was confirmed that the recombinant viruses were attenuated, through that the plaque size of the designed recombinant viruses was smaller than the wild type virus. Among them, the plaque size of the recombinant viruses prepared in Examples 1, 2, 3, 4, 6, 7, 9 was confirmed to be particularly small.

### 7. In vivo attenuation test

The 10 kinds of viruses were administered by concentration of 10¹ ~ 10⁷ pfu/mouse through an IN (Intranasal), IM (intramuscular) or IP (intraperitoneal) route of BALB/c mice, Type I KO mice or cotton rats. On day 1~7, the viruses were detected in blood or lung using q-PCR or plaque assay, and various changes such as the body weight, mortality, lung inflammation and the like were measured for 2 weeks. As a result, it was confirmed that the mutant viruses, the vaccine strains were attenuated compared to the wild type virus.

### 8. Virus stability test

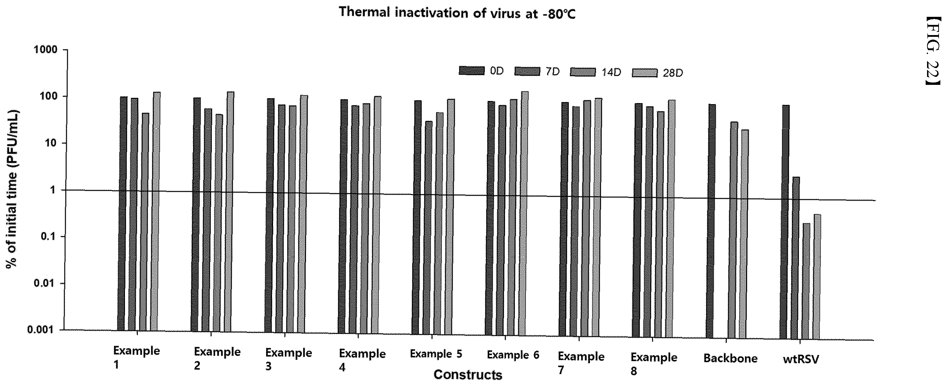

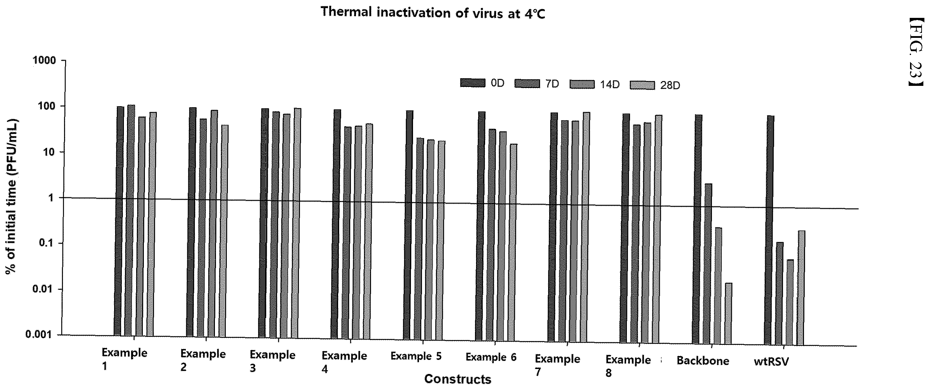

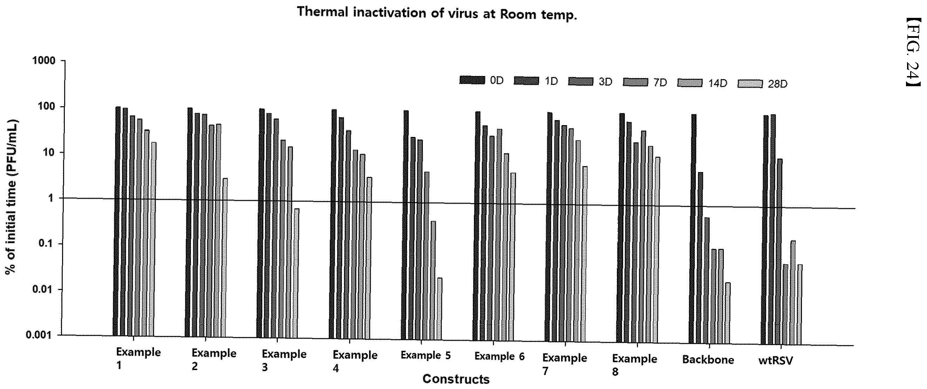

A total of 10 kinds of viruses including the wild type RSV and RSV backbone strain were stored at -80°C, 4°C, Room temp. for 28 days. The sample stored by each temperature condition was infected into verso cells, and through plaque assay, changes in titer depending on the temperature condition were analyzed. The result was shown in FIGs. 22 to 24.

As could be confirmed in FIGs. 22 to 24, it was confirmed that the stability of the mutant viruses increased compared to the wild type.

### 9. Immunogenicity test

The total antibody titer was measured using a total of 11 kinds of viruses including Examples 1-9. The 10 kinds of viruses were intranasally (In) inoculated into BALB/c, female 7w mice (n=5 per group), and the virus was inoculated once at a concentration of 1×10⁴ pfu/mouse (35ul/mouse) in case of the low-dose group, and at a concentration of 1×10⁵ pfu/mouse in case of the high-dose group, respectively. The vehicle control group was treated with the same schedule (35ul/mouse). In 14 days, 28 days and 40 days after the first inoculation, blood was collected and serum was separated through centrifugation. The total antibody titer was measured using the 14th day, and 28th day serum by ELISA analysis, and neutralizing antibodies were measured using the 40th day serum through plaque reduction neutralization test (PRNT).

### 9-1. Total antibody titer

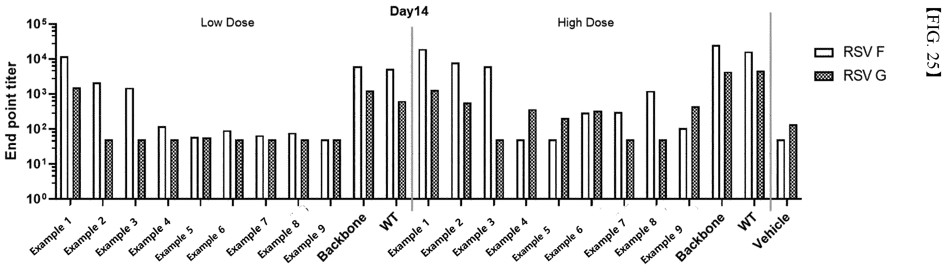

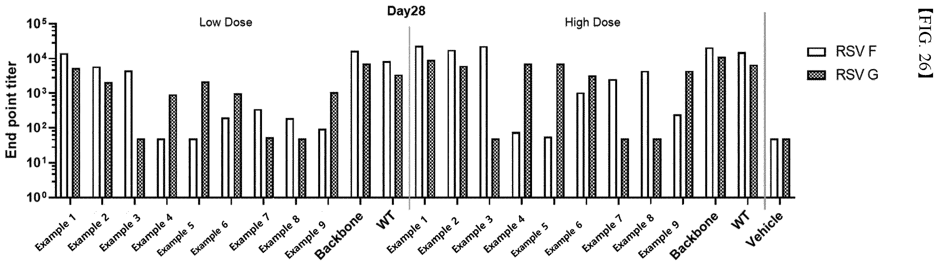

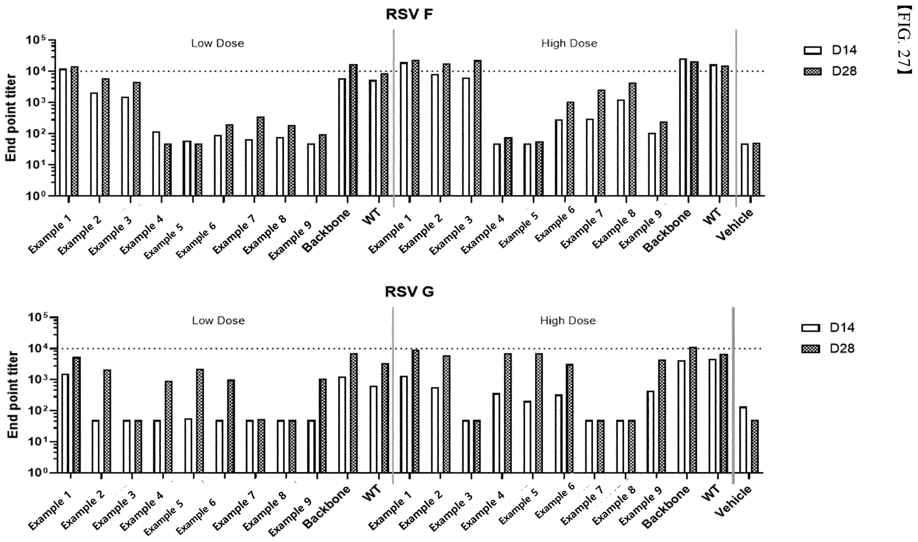

ELISA analysis of the mouse serum to which the recombinant virus of the present invention was inoculated was performed. As a result, as shown in FIGs. 25 to 27, it was confirmed that an IgG antibody specific to the administered RSV antigen was formed in all the groups except for Mock. Specifically, in case of the anti-RSV F IgG in 14 days after the first inoculation (Day 14), a difference of 4 times on average and 16 times at maximum in the end point titer was shown in the high-dose group compared to the low-dose group, and in case of the anti-RSV G IgG, a difference of 4.7 times on average and 11 times at maximum in the end point titer was shown in the high-dose group compared to the low-dose group (FIG. 25). In case of the anti-RSV F IgG in 28 days after the first inoculation (Day 28), a difference of 4.8 times on average and 23 times at maximum in the end point titer was shown in the high-dose group compared to the low-dose group, and in case of the anti-RSV G IgG, a difference of 2.6 times on average and 7.6 times at maximum in the end point titer was shown in the high-dose group compared to the low-dose group (FIG. 27).

### 9-2. Neutralizing antibody titer

PRNT analysis was performed using the Day 40 serum of the mice inoculated with the recombinant virus of the present invention. After diluting the recovered serum by 4, 8, 16 and 32 times, respectively, it was reacted with the wild type virus, and then infected to vero cells. On Day 3 after infection, plaque assay was performed to calculate an IC₅₀ value by each group.

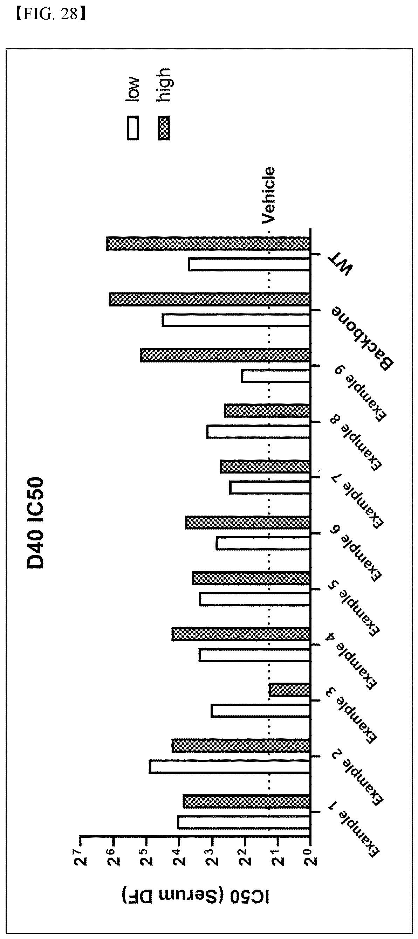

As a result, as shown in FIG. 28, it was confirmed that antibodies neutralizing virus infection were sufficiently formed in all the immune groups.

### 10. Immune effect test

Following the 9. Immunogenicity test, RSV A was challenged to the mouse nasal cavity at a concentration of 5×10⁵pfu/mouse after 35 days of the first inoculation. On Day 1~5, the virus was detected in the lung using plaque assay, and the body weight and clinical symptoms were measured for 5 days.

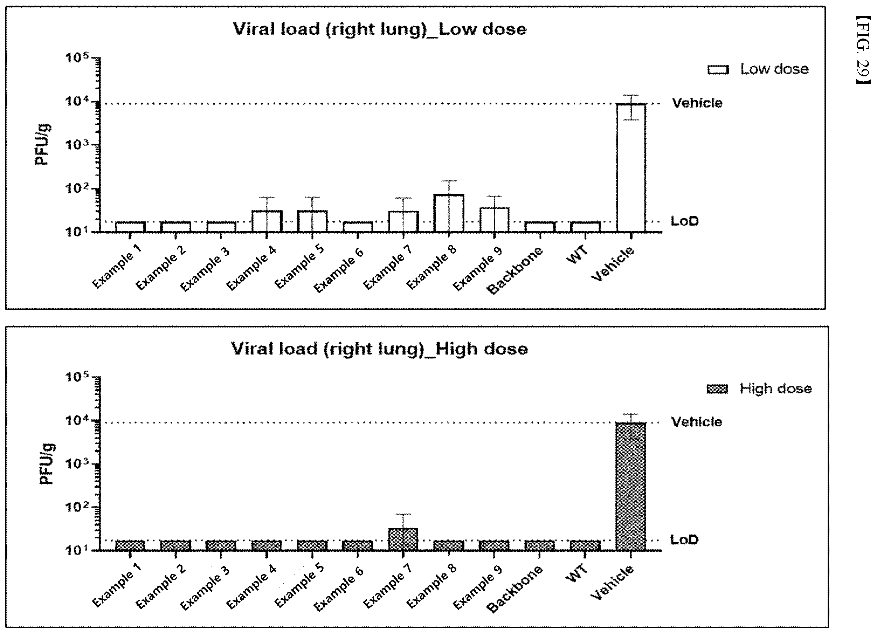

On Day 40, mice were sacrificed, and lung tissue was recovered, and this was homogenized, and residual virus was analyzed by plaque assay. As a result, as could be confirmed in FIG. 29, it was confirmed that the recombinant virus showed an effect of reducing the RSV A viral load in the lung in both the low concentration group and high concentration group. Through this, it was confirmed that virus infection was effectively inhibited in all the immune groups. In addition, it was confirmed that the body weight was not significantly reduced in all the immune groups, and even if it decreased by about 1~5%, an aspect of rapid recovery was confirmed, and no special clinical symptoms were observed.

### INDUSTRIAL APPLICABILITY

The present invention can be used as a pharmaceutical composition, preferably, vaccine for preventing infection of an RSV (respiratory syncytial virus).

## Claims

1. A recombinant attenuated respiratory syncytial virus comprising a nucleic acid encoding an F protein of a stabilized pre-fusion respiratory syncytial virus (RSV) or its analogue, variant, or fragment;
or
a nucleic acid encoding a G protein of vesicular stomatitis Indiana virus (VSV) or its analogue, variant, or fragment.

2. The recombinant attenuated respiratory syncytial virus according to claim 1, wherein the G protein of vesicular stomatitis Indiana virus (VSV) consists of the amino acid sequence of SEQ ID NO: 2.

3. The recombinant attenuated respiratory syncytial virus according to claim 1, wherein the recombinant attenuated respiratory syncytial virus (RSV) comprising a nucleic acid encoding a G protein of vesicular stomatitis Indiana virus (VSV) or its analogue, variant, or fragment is,
**characterized in that** a nucleic acid encoding at least one selected from the group consisting of SH, G, and F proteins of the RSV is further i) deleted, or ii) substituted with another nucleic acid, or iii) any one of nucleic acids encoding the proteins is deleted, and the others are substituted with another nucleic acid.

4. The recombinant attenuated respiratory syncytial virus according to claim 3, comprising an amino acid encoded by the nucleic acid sequence of SEQ ID NO: 11 or SEQ ID NO: 14, in case of the ii).

5. The recombinant attenuated respiratory syncytial virus according to claim 3, comprising an amino acid encoded by the nucleic acid sequence of SEQ ID NO: 12 or SEQ ID NO: 13, in case of the iii).

6. The recombinant attenuated respiratory syncytial virus according to claim 1,
wherein the F protein of a stabilized pre-fusion respiratory syncytial virus (RSV) has a mutation on at least one of furin cleavage sites of the F protein, and
SEQ ID NO: 55, the corresponding peptide of furin cleavage site II of an F gene, which is the 106-109th amino acid sequence of the F protein, is modified into the peptide of SEQ ID NO: 56, or SEQ ID NO:57, the corresponding peptide of furin cleavage site I of an F gene, which is the 133~136th amino acids, is substituted with SEQ ID NO: 58.

7. The recombinant attenuated respiratory syncytial virus according to claim 1,
wherein the F protein of a stabilized pre-fusion respiratory syncytial virus (RSV) is **characterized by** introducing a mutation that structurally stabilizes the F protein (D486L/E487L/F488W).

8. The recombinant attenuated respiratory syncytial virus according to claim 4, wherein the virus is one in which genes encoding NS1 and NS2 proteins comprised in the virus are further substituted, and
the antigenomic cDNA of the substituted genes consists of the nucleotide sequences represented by SEQ ID NOs: 32 and 33, respectively.

9. An isolated polynucleotide molecule comprising a nucleic acid encoding an F protein of a stabilized pre-fusion respiratory syncytial virus (RSV) or its analogue, variant, or fragment;
or
a nucleic acid encoding a G protein of vesicular stomatitis Indiana virus (VSV) or its analogue, variant, or fragment.

10. The polynucleotide molecule according to claim 9, wherein the nucleic acid is antigenomic cDNA or RNA.

11. The polynucleotide molecule according to claim 9, wherein the isolated polynucleotide molecule is cDNA consisting of a polynucleotide represented by at least one sequence selected from the group consisting of SEQ ID NOs: 6 to 16.

12. A vector comprising the polynucleotide molecule of claim 9.

13. A cell comprising the polynucleotide molecule of claim 9 or the vector of claim 12.

14. A method for producing a recombinant attenuated respiratory syncytial virus comprising transfecting the vector of claim 12 into a host cell;
culturing the cell or its culture for a sufficient time to allow virus replication; and
isolating a replicated recombinant respiratory syncytial virus.

15. A respiratory syncytial virus immunogenic composition comprising the recombinant attenuated RSV of any one claim of claim 1 to claim 8.

16. The immunogenic composition according to claim 15, wherein the immunogenic composition is a vaccine.

17. The immunogenic composition according to claim 16, wherein the vaccine is a live vaccine.

18. The immunogenic composition according to claim 15, wherein the immunogenic composition further comprises at least one selected from the group consisting of a carrier, a diluent, an excipient and an adjuvant.

19. The immunogenic composition according to claim 15, wherein the immunogenic composition is administered through an intranasal, intratracheal, intramuscular, intradermal or subcutaneous route.

20. A pharmaceutical composition for preventing or treating infection of a respiratory syncytial virus comprising the immunogenic composition of claim 15.

21. A method for inducing an immune response against an RSV in a subject, comprising administering a pharmaceutical composition comprising the recombinant attenuated RSV of any one claim of claim 1 to claim 8; and a pharmaceutically acceptable carrier in an effective amount to produce immune into a subject in need thereof.

22. A method for preventing infection by an RSV by inducing a neutralizing antibody response against a respiratory syncytial virus in a subject, comprising administering a pharmaceutical composition comprising the recombinant attenuated RSV of any one claim of claim 1 to claim 8; and a pharmaceutically acceptable carrier into a subject in need thereof.

23. A use of the recombinant attenuated RSV of any one claim of claim 1 to claim 8, for inducing an immune response against a respiratory syncytial virus in a subject.

24. A use of the recombinant attenuated RSV of any one claim of claim 1 to claim 8, or the polynucleotide molecule of any one claim of claim 9 to claim 11, for preparing a drug for preventing or treating infection by an RSV.
